# EUROPEAN PATENT APPLICATION

(11) **EP 2 085 029 A1**
(43) Date of publication of application: **05.08.2009**
(21) Application number: 09250104.8
(22) Date of filing: 16.01.2009
(51) Int. Cl.: A61B 5/145

(54) **Analyte testing method and system**

(30) Priority: 18.01.2008 US 21995 P; 18.01.2008 US 22042 P; 06.06.2008 US 59473 P
(71) Applicant: Lifescan Scotland Limited, Inverness-shire IV2 3ED (GB)
(72) Inventor: Orr, Allan, Inverness, Inverness-shire IV2 5RH (GB); Young, Stan, Nort Kessock, IV1 3XN (GB); Chiba, Mel, San Jose, California 95124 (US); Shankey, Mark, Dunfermline KY11 8GR (GB); Petkov, Anton, Zug 6300 (CH)
(74) Representative: Tunstall, Christopher Stephen

(57) **Abstract**

Described and illustrated herein is an exemplary method of operating an analyte measurement device having a display, user interface, processor, memory, and user interface buttons. Such method can be achieved by measuring an analyte with the analyte measurement device, displaying a value representative of the analyte, prompting a user to select a flag to associate the flag with the value, and pressing only one of the user interface buttons once to store the flag with the value in the memory of the analyte measurement device. In one embodiment, the testing device is a glucose meter and the analyte being tested is glucose.

## Description

### Background

Glucose monitoring is a fact of everyday life for diabetic individuals. The accuracy of such monitoring can significantly affect the health and ultimately the quality of life of the person with diabetes. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. There are a number of electronic devices currently available which enable an individual to test the glucose level in a small sample of blood. One such glucose meter is the OneTouch® Profile ™ glucose meter, a product which is manufactured by Lifescan.

In addition to glucose monitoring, diabetic individuals often have to maintain tight control over their lifestyle, so that they are not adversely affected by, for example, irregular food consumption or exercise. In addition, a physician dealing with a particular diabetic individual requires detailed information on the lifestyle of the individual to provide effective treatment or modification of treatment for controlling diabetes. Currently, one of the ways of monitoring the lifestyle of an individual with diabetes has been for the individual to keep a paper logbook of their lifestyle. Another way is for an individual to simply rely on remembering facts about their lifestyle and then relay these details to their physician on each visit.

The aforementioned methods of recording lifestyle information are inherently difficult, time consuming, and possibly inaccurate. Paper logbooks are not necessarily always carried by an individual and may not be accurately completed when required. Such paper logbooks are small and it is therefore difficult to enter detailed information requiring detailed descriptors of lifestyle events. Furthermore, an individual may often forget key facts about their lifestyle when questioned by a physician who has to manually review and interpret information from a hand-written notebook. There is no analysis provided by the paper logbook to distil or separate the component information. Also, there are no graphical reductions or summary of the information. Entry of data into a secondary data storage system, such as a database or other electronic system, requires a laborious transcription of information, including lifestyle data, into this secondary data storage. Difficulty of data recordation encourages retrospective entry of pertinent information that results in inaccurate and incomplete records.

Moreover, a diabetic individual often has to keep a plurality of devices on their person for diagnosis and treatment, for example both glucose level monitoring equipment and medication. Hence, having to carry paper records of their lifestyle is an added unwanted burden and entry of data therein is very time consuming.

There currently exist a number of portable electronic devices that can measure glucose levels in an individual and store the levels for recalling or uploading to another computer for analysis. One such device is the Accu-Check^{™} Complete^{™} System from Roche Diagnostics, which provides limited functionality for storing lifestyle data. However, the Accu-Check^{™} Complete^{™} System only permits a limited selection of lifestyle variables to be stored in a meter. There is a no intelligent feedback from values previously entered into the meter and the user interface is unintuitive for an infrequent user of the meter.

### Summary of the Disclosure

Applicants have recognized a need for an electronic device for logging and analyzing lifestyle data, which does not increase the number of devices an individual has to keep on their person. Such device must be also more intuitive and easier to use than other devices, thereby encouraging an individual to record information related to their lifestyle. Lifestyle data should be taken to mean any quantifiable information, which might affect or represent an individual's physical condition. Examples of lifestyle data are food consumption, physical exertion (e.g. exercise), medication intake and health checks performed on the individual.

In view of the foregoing and in accordance with one aspect, there is provided a method of operating an analyte measurement device having a display, user interface, processor, memory and user interface buttons. The method can be achieved by measuring an analyte with the analyte measurement device; displaying a value representative of the analyte; prompting a user to select a flag to associate the flag with the value; and pressing only one of the user interface buttons once to store the flag with the value in the memory of the device.

In an embodiment, the prompting may include repetitively flashing on the display an icon representative of the one of the user interface buttons to prompt a selection of such user interface button.

In an embodiment, the icon includes an icon selected from a group consisting of a first triangle and a second triangle having a smaller area than the first triangle.

In an embodiment, the method further includes disabling all of the user interface buttons except for one of the user interface buttons.

In an embodiment, user interface buttons include an up button, a down button, and an enter button.

In an embodiment, user selectable flags include a before meal flag or an after meal flag.

In an embodiment, user selectable flags include an after meal flag.

In an embodiment, the prompting includes always prompting a user whenever a measuring step has been completed.

In an embodiment, the prompting includes prompting a user whenever a measuring step indicates that an analyte value is outside a predetermined range.

In an embodiment, the pressing includes storing in memory the date and time at the completion of the measuring step along with the selected flag.

In an embodiment, the analyte measurement device includes a glucose meter.

In an embodiment, measuring includes inserting a test strip into a strip port provided by the measurement device; and depositing a blood sample on a testing portion of the test strip without entering a calibration parameter for the test strip.

In an embodiment, the measuring includes inserting a test strip into a strip port provided by the measurement device; inputting a calibration parameter for the test strip via the user interface buttons of the device; and depositing a blood sample on a testing portion of the test strip.

In an embodiment, the inserting includes turning on the measurement device when the strip is fully inserted into the strip port.

In an embodiment, one of a plurality of user selectable flags is selected from a group consisting essentially of at least one of a comment title, a plurality of comments, comment page number, no comment, not enough food, too much food, mild exercise, strenuous exercise, medication, stress, illness, hypoglycemic state, menses, vacation, and combinations thereof.

In an embodiment, a plurality of menus is displayed.

In an embodiment, one of a plurality of menus includes a prompt for last result, all results, result average, and set up.

In an embodiment, a plurality of menus includes a display of a prompt for all results average, before meal average, after meal average.

In view of the foregoing and in accordance with another aspect, there is provided a method of operating an analyte measurement device having a display, user interface, processor, memory and user interface buttons. The method can be achieved by measuring an analyte with the analyte measurement device; displaying a value representative of the analyte; prompting a user to select a flag to associate the flag with the value whenever the measuring is completed; ignoring activation of any of the user interface buttons except for a selected button; and associating the value with the flag upon activation of the selected button in the memory of the device.

In an embodiment, the prompting includes repetitively flashing on the display an icon representative of the selected user interface buttons to prompt a selection of such user interface button.

These and other embodiments, features and advantages will become apparent to those skilled in the art when taken with reference to the following more detailed description of the invention in conjunction with the accompanying drawings that are first briefly described.

### Brief Description of the Figures

The accompanying drawings, which are incorporated herein and constitute part of this specification, illustrate presently preferred embodiments of the invention, and, together with the general description given above and the detailed description given below, serve to explain features of the invention (wherein like numerals represent like elements), of which:

Figure 1 is an exemplary plan view of an analyte measurement device, according to an embodiment.

Figure 2 is an exemplary block diagram illustrating the principal internal components of an analyte measurement device, according to an embodiment.

Figure 3 is an exemplary flow chart illustrating a method of operating an analyte measurement device, according to an embodiment.

Figure 4 is an exemplary flow chart illustrating a method of operating an analyte measurement device when only a single user interface button on the analyte measurement device is active, according to an embodiment.

Figure 5 is an exemplary flow chart illustrating a method of operating an analyte measurement device where a user is prompted or queried when an analyte value is outside a predetermined range, according to an embodiment.

Figure 6 is an exemplary flow chart illustrating a method of operating an analyte measurement device where a flag, an analyte value, and the date and time of a measurement are stored in the memory of the analyte measurement device, according to an embodiment.

Figure 7 is an exemplary flow chart illustrating a method of operating an analyte measurement device after inserting a test strip into a strip port in the analyte measurement device, according to an embodiment.

Figure 8 is an exemplary flow chart illustrating a method of operating an analyte measurement device after inserting a test strip into a strip port in the analyte measurement device and either entering or confirming calibration parameters of the test strip, according to an embodiment.

Figure 9 is an exemplary flow chart illustrating a method of operating an analyte measurement device after inserting a test strip into a strip port in the analyte measurement device thereby turning the analyte measurement device on, according to an embodiment.

Figure 10 is an exemplary flow chart illustrating a method of operating an analyte measurement device where all but one user interface buttons are ignored, according to an embodiment.

Figure 11 is an exemplary flow chart illustrating a method of operating an analyte measurement device and actions taken by the analyte measurement device, according to an embodiment.

Figure 12 illustrates a series of user interface screens used in a method of operating an analyte measurement device, according to an embodiment.

### Detailed Description of the Figures

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

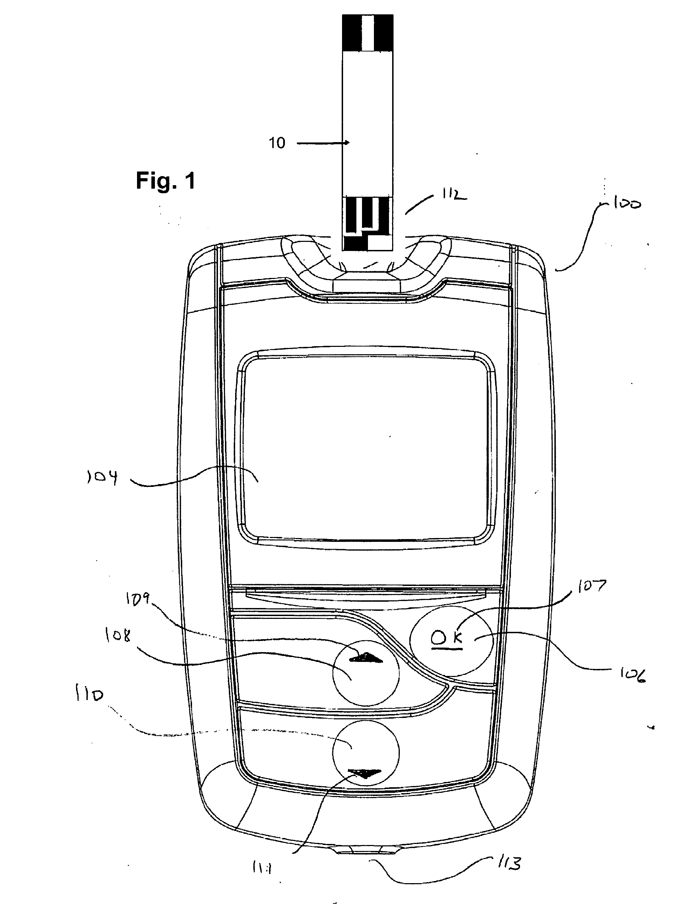

Figure 1 illustrates an analyte measurement device 100, for testing glucose levels in the blood of an individual. Analyte measurement device 100 may include user interface buttons (106, 108, 110) for entry of data, navigation of menus, and execution of commands. Data can include values representative of analyte concentration, and/or information, which are related to the everyday lifestyle of an individual. Information, which is related to the everyday lifestyle, can include food intake, medication use, the occurrence of health check-ups and general health condition and exercise levels of an individual. Analyte measurement device 100 also may include display 104. Display 104 can be used to report measured glucose levels, and to facilitate entry of lifestyle related information.

Analyte measurement device 100 may include first user interface button 106, second user interface button 108, and third user interface button 110. User interface buttons 106, 108, and 110 facilitate entry and analysis of data stored in the testing device, enabling a user to navigate through the user interface displayed on display 104. User interface buttons 106, 108, and 110 include first marking 107, second marking 109, and third marking 111, which help in correlating user interface buttons to characters on display 104.

Analyte measurement device 100 can be turned on by inserting a test strip 10 into a strip port 112, by pressing and briefly holding first user interface button 106, or when data traffic is detected across data port 113. Analyte measurement device 100 can be switched off by removing the test strip 10, pressing and briefly holding first user interface button 106, navigating to and selecting a meter off option from a main menu screen, or by not pressing any buttons for a predetermined time. Display 104 can optionally include a backlight.

Data port 113 accepts a suitable connector attached to a connecting lead, thereby allowing analyte measurement device 100 to be linked to an external device such as a personal computer. Data port 113 can be any port that allows for transmission of data (serial or parallel) such as, for example, serial or parallel port in wired or wireless form. A personal computer, running appropriate software, allows entry and modification of set-up information (e.g. the current time, date, and language), and can perform analysis of data collected by analyte measurement device 100. In addition, the personal computer may be able to perform advanced analysis functions, and/or transmit data to other computers (i.e. over the internet) for improved diagnosis and treatment. Connecting analyte measurement device 100 with a local or remote computer facilitates improved treatment by health care providers.

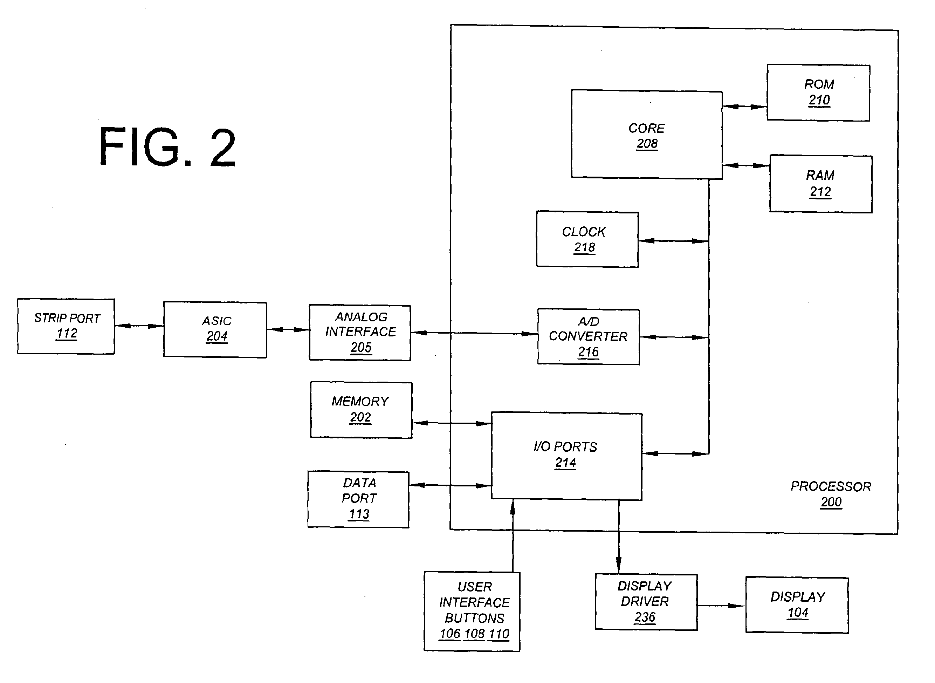

Referring to Fig. 2, an exemplary internal layout of analyte measurement device 100 is shown. Analyte measurement device 100 may include a processor 200, which in some embodiments described and illustrated herein is a 32-bit RISC microcontroller. In other embodiments described and illustrated herein, processor 200 is selected preferably from the MSP 430 family of ultra-low power microcontrollers manufactured by Texas Instruments of Dallas, Texas. The processor can be bi-directionally connected via I/O ports 214 to memory 202, which in some embodiments described and illustrated herein is an EEPROM. Also connected to processor 200 via I/O ports 214 are the data port 113, the user interface buttons 106, 108, and 110, and a display driver 236. Data port 113 can be connected to processor 200, thereby enabling transfer of data between memory 202 and an external device, such as a personal computer. User interface buttons 106, 108, and 110 are directly connected to processor 200. Processor 200 controls display 104 via display driver 236.

In embodiments described and illustrated herein, analyte measurement device 100 may include an Application Specific Integrated Circuit (ASIC) 204, providing electronic circuitry used in measurements of glucose level in blood that has been applied to a test strip 10 inserted into strip port 112. Analog voltages can pass to and from ASIC 204 by way of analog interface 205. Analog signals from analog interface 205 can be converted to digital signals by A/D converter 216. Processor 200 further includes core 208, ROM 210 (containing computer code), RAM 212, and clock 218. In one embodiment, the processor 200 is configured (or programmed) to disable all of the user interface buttons except for a single button upon a display of an analyte value by the display unit such as, for example, during a time period after an analyte measurement. In an alternative embodiment, the processor 200 is configured (or programmed) to ignore any input from all of the user interface buttons except for a single button upon a display of an analyte value by the display unit.

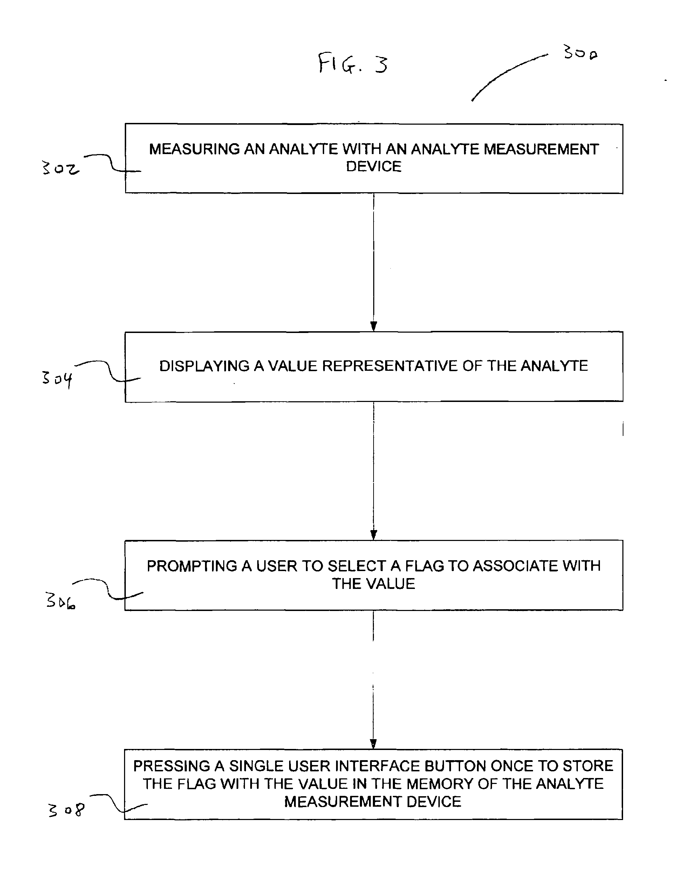

Figure 3 is an exemplary flow chart illustrating a method of operating an analyte measurement device, according to an embodiment described and illustrated herein. Method 300 includes steps 302, 304, 306, and 308. In step 302, an analyte measuring device measures an analyte. In step 304, the analyte measuring device displays a value representative of the analyte. In step 306, the analyte measuring device prompts the user to select a flag to associate with the displayed value. In step 308, a single user interface button is pressed once, causing the flag and the displayed value to be stored in the memory of the analyte measurement device. In any embodiment described and illustrated herein, the analyte measurement device may include a display, a user interface, a processor, a memory and user interface buttons. In any embodiments described and illustrated herein, prompting may include repetitively flashing on the display an icon representative of one of the user interface buttons to prompt a selection of such user interface button. In any embodiment described and illustrated herein, the icon may be selected from a group consisting of a first triangle and a second triangle having a smaller area than the first triangle.

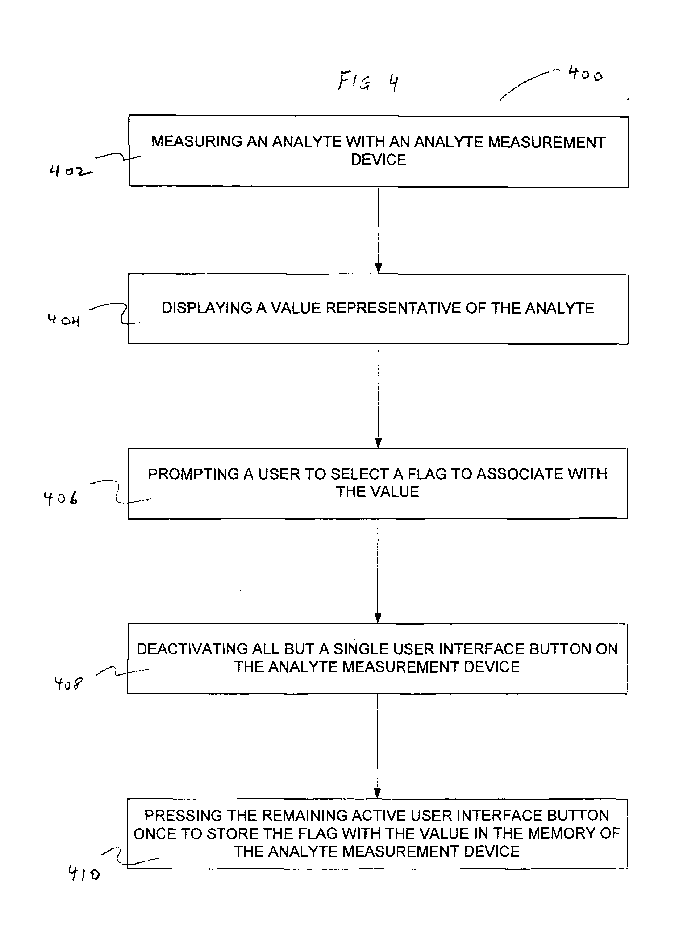

Figure 4 is an exemplary flow chart illustrating a method of operating an analyte measurement device when only a single user interface button on the analyte measurement device is active, according to an embodiment described and illustrated herein. Method 400 includes steps 402, 404, 406, 408, and 410. In step 402, an analyte measuring device measures an analyte. In step 404, the analyte measuring device displays a value representative of the analyte. In step 406, the analyte measuring device prompts the user to select a flag to associate with the displayed value. In step 408, the analyte measuring device deactivates all but a single user interface button. In step 410, the active user interface button is pressed once, causing the flag and the displayed value to be stored in the memory of the analyte measurement device. In any embodiment described and illustrated herein, user interface buttons may include an "up" button, a "down" button, and an "enter" or "OK" button. In any embodiment described and illustrated herein, user selectable flags may include a before meal flag or an after meal flag. In any embodiment described and illustrated herein, prompts may be used whenever a measuring step has been completed.

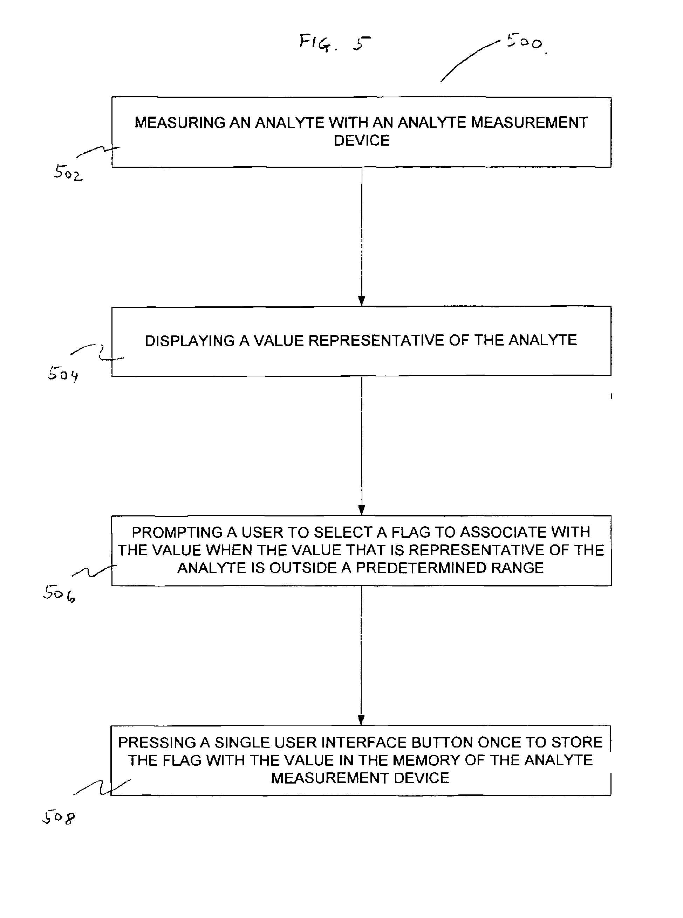

Figure 5 is an exemplary flow chart illustrating a method of operating an analyte measurement device where a user is prompted or queried when an analyte value is outside a predetermined range, according to an embodiment described and illustrated herein. Method 500 includes steps 502, 504, 506, and 508. In step 502, an analyte measuring device measures an analyte. In step 504, the analyte measuring device displays a value representative of the analyte. In step 506, the analyte measuring device prompts the user to select a flag to associate with the displayed value when the displayed value is outside a predetermined range. In step 508, a single user interface button is pressed once, causing the flag and the displayed value to be stored in the memory of the analyte measurement device.

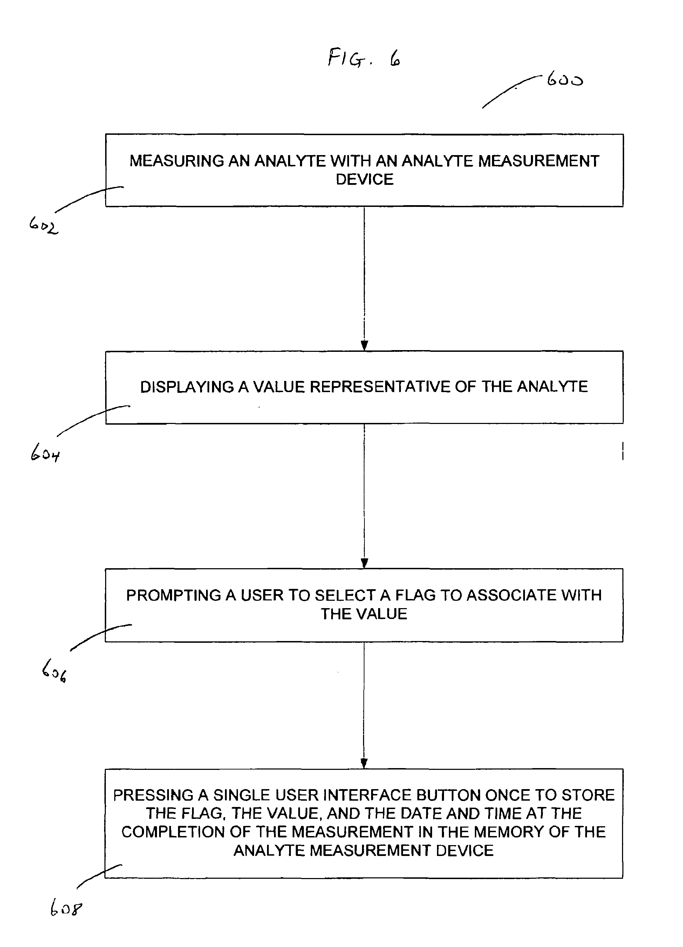

Figure 6 is an exemplary flow chart illustrating a method of operating an analyte measurement device where a flag, an analyte value, and the date and time of a measurement are stored in the memory of the analyte measurement device, according to an embodiment described and illustrated herein. Method 600 includes steps 602, 604, 606, and 608. In step 602, an analyte measuring device measures an analyte. In step 604, the analyte measuring device displays a value representative of the analyte. In step 606, the analyte measuring device prompts or queries the user to select a flag to associate with the displayed value. In step 608, a single user interface button is pressed once, causing the flag, the displayed value, and the date and time at the completion of the measurement to be stored in the memory of the analyte measurement device. In any embodiment described and illustrated herein, the analyte measuring device may include a glucose meter. As used herein, the term "prompting" may involve awaiting for an input by a user. The term "querying" may involve a displaying of a message and awaiting for an input by the user.

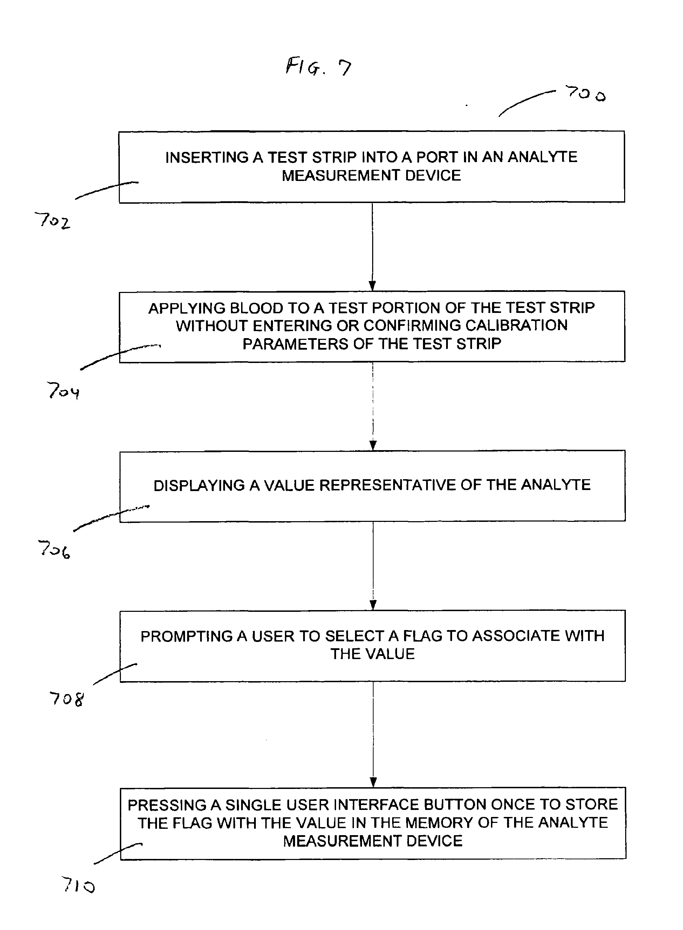

Figure 7 is an exemplary flow chart illustrating a method of operating an analyte measurement device after inserting a test strip 10 into a strip port 113 in the analyte measurement device, according to an embodiment described and illustrated herein. Method 700 includes steps 702, 704, 706, 708, and 710. In step 702, a test strip 10 is inserted into a strip port in an analyte measurement device. In step 704, blood is applied to a test portion (the portion distal from the strip port 112) of the test strip 10 without entering or confirming calibration parameters of the test strip 10. In step 706, the analyte measuring device displays a value representative of the analyte. In step 708, the analyte measuring device prompts the user to select a flag to associate with the displayed value. In step 710, a single user interface button is pressed once, causing the flag and the displayed value to be stored in the memory of the analyte measurement device. In any embodiment described and illustrated herein, measuring may include: inserting a test strip 10 into a strip port in the analyte measurement device, then depositing a sample of blood on a testing portion of the test strip 10 without entering a calibration parameter for the test strip 10.

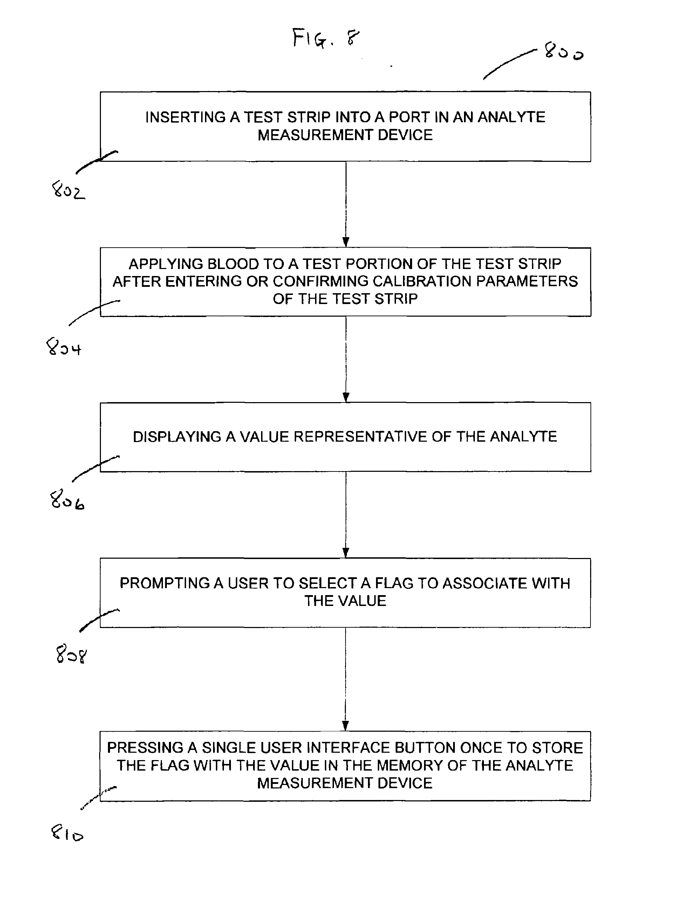

Figure 8 is an exemplary flow chart illustrating a method of operating an analyte measurement device after inserting a test strip 10 into a strip port in the analyte measurement device and either entering or confirming calibration parameters of the test strip 10, according to an embodiment described and illustrated herein. Method 800 includes steps 802, 804, 806, 808, and 810. In step 802, a test strip 10 is inserted into a strip port in an analyte measurement device. In step 804, blood is applied to a test portion of the test strip 10 after entering or confirming calibration parameters of the test strip 10. In step 806, the analyte measuring device displays a value representative of the analyte. In step 808, the analyte measuring device prompts the user to select a flag to associate with the displayed value. In step 810, a single user interface button is pressed once, causing the flag and the displayed value to be stored in the memory of the analyte measurement device. In any embodiment described and illustrated herein, measuring may include: inserting a test strip 10 into a strip port in the measurement device; inputting a calibration parameter for the test strip 10 via the user interface buttons of the device; and depositing a blood sample on a testing portion of the test strip 10.

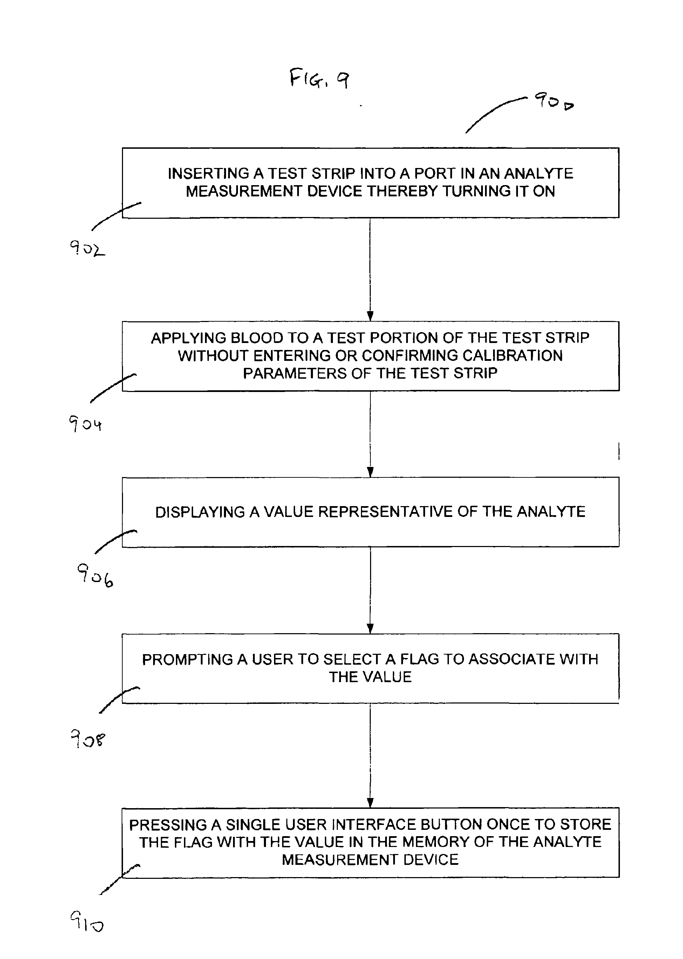

Figure 9 is an exemplary flow chart illustrating a method of operating an analyte measurement device after inserting a test strip 10 into a strip port in the analyte measurement device thereby turning the analyte measurement device on, according to an embodiment described and illustrated herein. Method 900 includes steps 902, 904, 906, 908, and 910. In step 902, a test strip 10 is inserted into a strip port in an analyte measurement device, thereby turning it on. In step 904, blood is applied to a test portion of the test strip 10 without entering or confirming calibration parameters of the test strip 10. In step 906, the analyte measuring device displays a value representative of the analyte. In step 908, the analyte measuring device prompts the user to select a flag to associate with the displayed value. In step 910, a single user interface button is pressed once, causing the flag and the displayed value to be stored in the memory of the analyte measurement device. In any embodiment described and illustrated herein, inserting may include turning on the measurement device when the strip is fully inserted into the strip port. In any embodiment described and illustrated herein, one of a plurality of user selectable flags may be selected from a group consisting essentially of at least one of a comment title, a plurality of comments, comment page number, no comment, not enough food, too much food, mild exercise, strenuous exercise, medication, stress, illness, hypoglycemic state, menses, vacation, and combinations thereof. In any embodiment described and illustrated herein, a plurality of menus may be displayed. In any embodiment described and illustrated herein, one of a plurality of menus may include a prompt for last result, all results, result average, and set up. In any embodiment described and illustrated herein, a plurality of menus may include a display of a prompt for all results average, before meal average, after meal average.

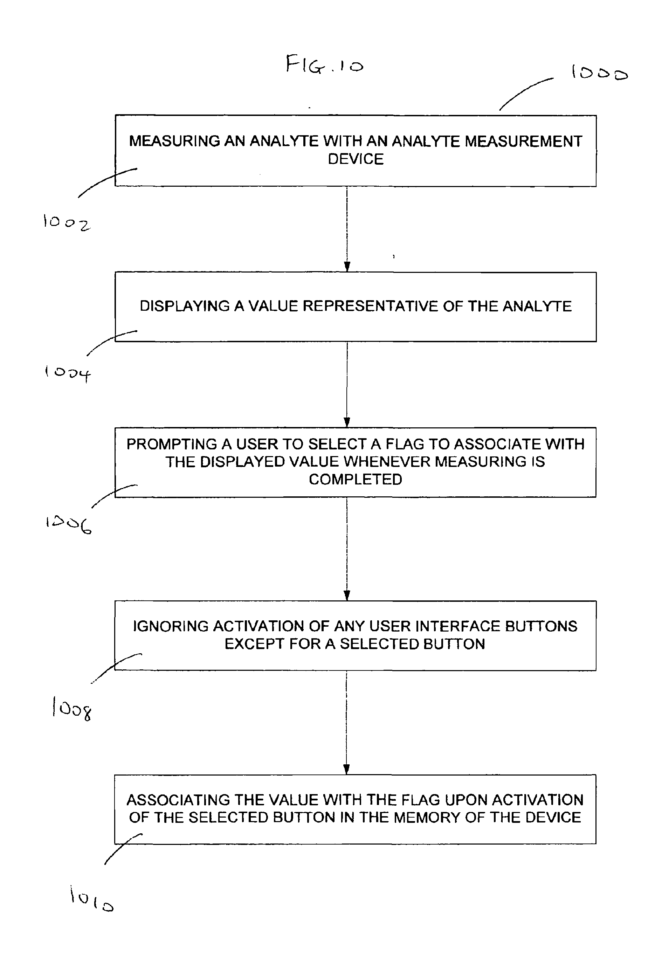

Figure 10 is an exemplary flow chart illustrating a method of operating an analyte measurement device where all but one user interface buttons are ignored, according to an embodiment described and illustrated herein. Method 1000 includes steps 1002, 1004, 1006, 1008, and 1010. In step 1002, an analyte measuring device measures an analyte. In step 1004, the analyte measuring device displays a value representative of the analyte. In step 1006, the analyte measuring device prompts the user to select a flag to associate with the displayed value whenever measuring is completed. In step 1008, the analyte measuring device ignores activation of all but a single user interface button. In step 1010, the single non-ignored user interface button is pressed once, causing the flag and the displayed value to be stored in the memory of the analyte measurement device. In any embodiment described and illustrated herein, prompting may include repetitively flashing on the display an icon representative of a single user interface button to prompt selection of the single user interface button.

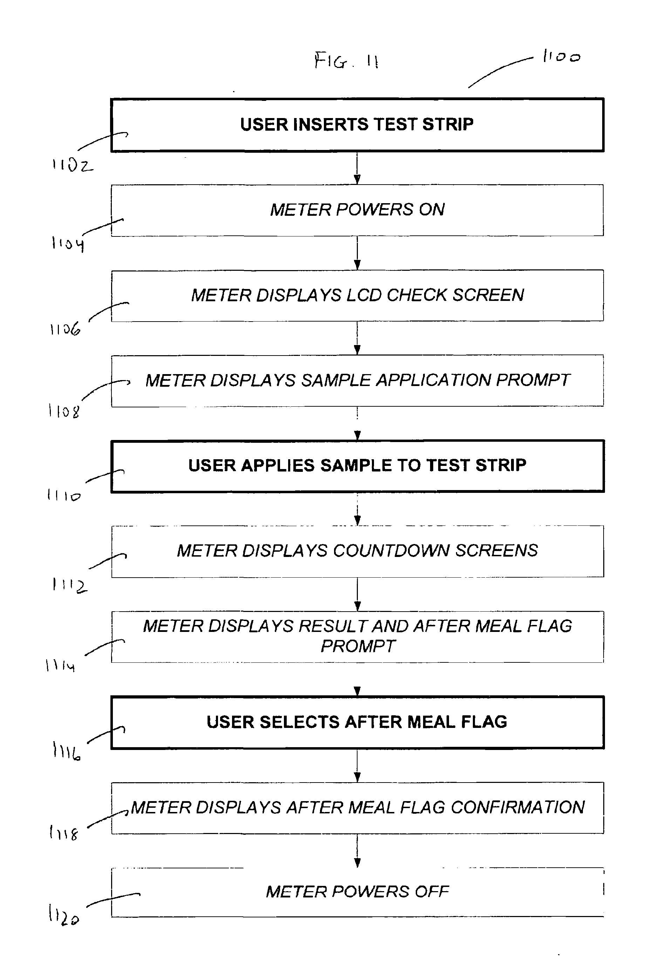

Figure 11 is an exemplary flow chart illustrating a method of operating an analyte measurement device and actions taken by the analyte measurement device, according to an embodiment described and illustrated herein. Method 1100 includes steps 1102, 1104, 1106, 1108, 1110, 1112, 1114, 1116, 1118, and 1120. In step 1102, a user inserts a test strip 10 into a strip port in an analyte measurement device. In step 1104, the analyte measuring device turns on. In step 1106, the analyte measuring device displays an LCD check screen. In step 1108, the analyte measuring device displays a sample application prompt. In step 1110, the user applies sample to the test strip 10. In step 1112, the analyte measuring device displays a series of countdown screens. In step 1114, the analyte measuring device displays a value representative of the analyte and prompts the user to select an after meal flag to associate with the displayed value. In step 1116, the user selects an after meal flag, causing the flag and the displayed value to be stored in the memory of the analyte measurement device. In step 1118, the analyte measurement device displays an after meal flag confirmation. In step 1120, the analyte measurement device turns off after a predetermined time, without interaction from the user.

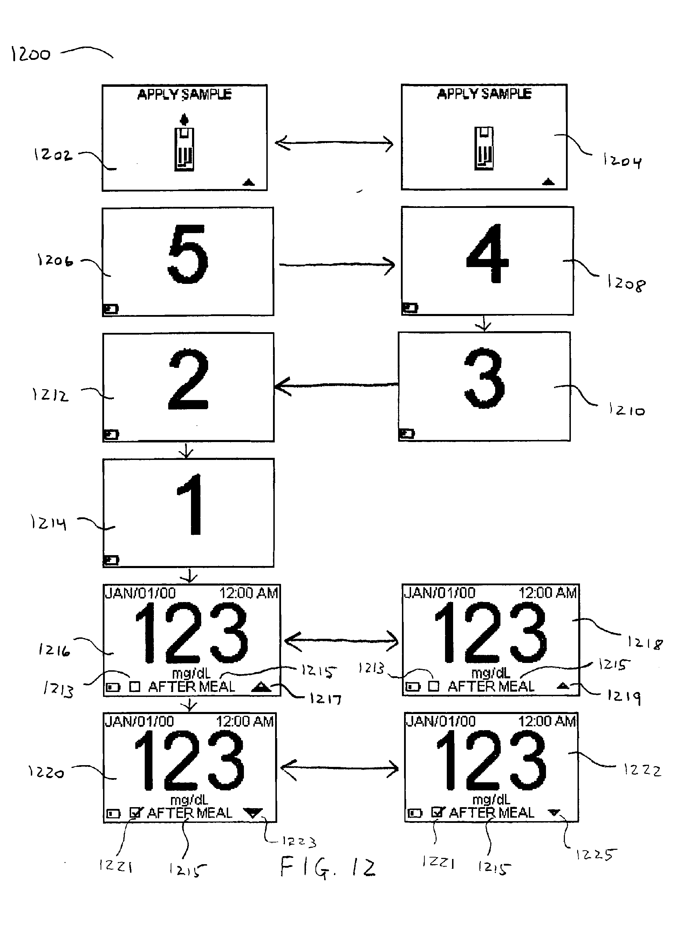

Figure 12 illustrates a series of user interface screens displayed during a method of operating an analyte measurement device, according to an embodiment described and illustrated herein. Method 1200 includes screens 1202, 1204, 1206, 1208, 1210, 1212, 1214, 1216, 1218, 1220, and 1222. In screens 1202 and 1204, the user is prompted to apply sample to a test strip 10 that has been inserted into a strip port in an analyte measurement device. In screen 1202 an icon symbolizing a drop of blood is displayed, while in screen 1204 there is no icon symbolizing a drop of blood. Screens 1202 and 1204 are alternated, creating the impression of a blinking drop of blood. Once sample is applied to the test strip 10, screens 1206, 1208, 1210, 1212, and 1214 are displayed, in succession. Screens 1206 through 1214 provide a countdown to result that is approximately 5 seconds in duration. In screens 1216 and 1218, the analyte measuring device displays a value representative of the analyte and prompts the user to select an after meal flag to associate with the displayed value. Screens 1216 and 1218 are alternated, creating the impression of blinking icons 1217 and 1219 next to the after meal prompt 1215. Blinking icons 1217 and 1219 draw attention to the after meal prompt 1215, and to a user interface button that includes a similar icon. Screens 1216 and 1218 include meal flag confirmation window 1213, which is empty when a meal flag has not been selected. In screens 1220 and 1222, the analyte measuring device displays a value representative of the analyte and indicates to the user that an after meal flag will be associated with the displayed value. Screens 1220 and 1222 are alternated, creating the impression of blinking icons 1223 and 1225 next to the after meal prompt 1215. Blinking icons 1223 and 1225 draw attention to the after meal prompt 1215, and to a user interface button that includes a similar icon. Screens 1220 and 1222 may include meal flag confirmation icon 1221, and is displayed after a user selects an after meal flag. If a user selects a user interface button that is correlated to blinking icons 1217 and 1219 when screen 1216 or 1218 is displayed, then screen 1220 or 1222 is displayed. If a user selects a user interface button that is correlated to blinking icons 1223 and 1225 when screen 1220 or 1222 is displayed, then screen 1216 or 1218 is displayed. In this way, the user can toggle between activating an after meal flag (as in screens 1220 and 1222), and deactivating an after meal flag (as in screens 1216 and 1218).

In conclusion, the testing device and methods described and illustrated herein significantly reduce obstacles associated with maintaining an accurate record of an individual's blood glucose testing and lifestyle. The various embodiments of the present invention is believed to promote frequent monitoring for diabetic individuals by providing a simple, efficient way of recording not only blood glucose levels, but other information which is likely to affect an individual's prognosis. By logging glucose and lifestyle information in the manner described herein, the testing device and methods described and illustrated herein provide an effective record keeping system.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the spirit of the disclosure or equivalent to the inventions found in the claims, it is the intent that this patent will cover those variations as well.

## Claims

1. A method of operating an analyte measurement device having a display, user interface, processor, memory and user interface buttons, the method comprising:
measuring an analyte in a biological fluid of a user with the analyte measurement device;
displaying a value representative of the analyte;
prompting or querying a user to select a flag to associate the flag with the value of the analyte; and
pressing only one of the user interface buttons once to store the flag with the value in the memory of the device.

2. The method of claim 1, in which the prompting comprises repetitively flashing on the display an icon representative of the one of the user interface buttons to suggest a selection of such user interface button.

3. The method of claim 2, in which the icon comprises a first triangle.

4. The method of claim 1, further comprising disabling all of the user interface buttons except for the one of the user interface buttons.

5. The method of claim 1, in which the user interface buttons comprise an up button, a down button and an enter button.

6. The method of claim 1, in which the user selectable flag comprises a flag selected from a group consisting of a before meal flag or an after meal flag.

7. The method of claim 6, in which the user selectable flag comprises an after meal flag.

8. The method of claim 1, in which the prompting comprises always prompting a user whenever a measuring step has been completed.

9. The method of claim 1, in which the prompting comprises prompting a user whenever a measuring step indicates that an analyte value is outside a predetermined range.

10. The method of claim 1, in which the pressing comprises storing in memory the date and time at the completion of the measuring step with the selected flag.

11. The method of claim 1, in which the analyte measurement device comprises a glucose meter.

12. The method of claim 1, in which the measuring comprises:
inserting a test strip into a strip port provided by the measurement device; and
depositing a blood sample on a testing portion of the test strip without entering a calibration parameter for the test strip.

13. The method of claim 1, in which the measuring comprises
inserting a test strip into a strip port provided by the measurement device;
inputting a calibration parameter for the test strip via the user interface buttons of the device; and,
depositing a blood sample on a testing portion of the test strip.

14. The method of claim 1, in which the inserting comprises turning on the measurement device when the strip is fully inserted into the strip port.

15. The method of claim 4, in which the user selectable flag is selected from a group consisting essentially of at least one of a comment title, a plurality of comments, comment page number, no comment, not enough food, too much food, mild exercise, strenuous exercise, medication, stress, illness, hypoglycemic state, menses, vacation, and combinations thereof.

16. The method of claim 4, further comprising selecting a plurality of menus to be displayed.

17. The method of claim 16, in which one of the plurality of menus comprises a prompt for last result, all results, result average, and set up.

18. The method of claim 17, in which another of the plurality of menus comprises a display of a prompt for all results average, before meal average, and after meal average.

19. A method of operating an analyte measurement device having a display, user interface, processor, memory and user interface buttons, the method comprising:
measuring an analyte in a biological fluid of the user with the analyte measurement device;
displaying a value representative of the analyte;
prompting a user to select a flag to associate the flag with the value of the analyte whenever the measuring is completed;
ignoring activation of any of the user interface buttons except for a selected button; and
associating the value with the flag upon activation of the selected button in the memory of the device.

20. The method of claim 19, in which the prompting comprises repetitively flashing on the display an icon representative of the selected user interface buttons to suggest a selection of such user interface button.
